# EUROPÄISCHE PATENTANMELDUNG

(11) **EP 1 574 164 A1**
(43) Veröffentlichungstag der Anmeldung: **14.09.2005**
(21) Anmeldenummer: 05004618.4
(22) Anmeldetag: 03.03.2005
(51) Int. Cl.: A61B 5/00, G06F 19/00, G08C 17/00

(54) **Patientenüberwachungsvorrichtung**

(30) Priorität: 10.03.2004 DE 102004012042
(71) Anmelder: Smiths Medical Deutschland GmbH, 85614 Kirchseeon (DE)
(72) Erfinder: Beck, Bernd, 72414 Rangendingen (DE)
(74) Vertreter: von Bülow, Tam

(57) **Zusammenfassung**

Die Patientenüberwachungsvorrichtung mit mindestens einem patientenseitigen Meßwandler (11) hat eine patientenseitige Sendeeinrichtung (1) und eine davon entfernte Empfangseinrichtung (2), an die eine Auswerteeinheit (3) angeschlossen ist. Die Sende- und Empfangseinrichtung (1, 2) haben je mindestens einen Speicher (18, 44). Im Speicher (18) der Sendeeinheit (1) ist ein eindeutiger unveränderbarer Identifikationscode gespeichert. Die Empfangseinheit (2) hat eine Tastatur, über die ein Identifikationscode in deren Speicher (44) eingebbar ist. Eine Funkverbindung (4) zwischen Sende- und Empfangseinheit (1, 2) wird nur dann aufgebaut, wenn die beiden gespeicherten Identifikationscodes identisch sind. Bei Unterbrechung der Funkverbindung für eine vorgegebene Zeitdauer wird der Identifikationscode in der Empfangseinheit (2) automatisch gelöscht, und ein neuer Verbindungsaufbau ist erst nach Neueingabe des entsprechenden Identifikationscodes an der Empfangseinheit (2) möglich. Die Funkverbindung arbeitet nach dem Bluetooth-Protokoll.

## Beschreibung

Die vorliegende Erfindung bezieht sich auf eine Patientenüberwachungsvorrichtung gemäß dem Oberbegriff des Patentanspruches 1.

Eine solche Vorrichtung ist aus der DE 198 51 276 A1 bekannt. Dort sind zur invasiven Blutdruckmessung eines Patienten mehrere Druckmeßwandler vorgesehen, die an einer Halterung befestigbar sind. Meßdaten der Druckmeßwandler werden von der Halterung über eine einzige gemeinsame Signalübertragungseinrichtung an einen Monitor übertragen. Die Signalübertragungseinrichtung kann dabei ein Kabel oder eine drahtlose Signalübertragungseinrichtung sein, die aus einem Sender an der Halterung und an dem Empfänger am Monitor besteht.

Meßvorrichtungen zur Überwachung von Patientendaten, die diese drahtlos an einen Signalempfänger, wie z.B. einen Monitor, übertragen, sind auch aus der DE 100 09 591 A1, EP 0 672 381 B1, US 2001/0047125 A1, US 2002/0013614 A1, WO 01/45014 A1, WO 01/97907 A2 und WO 02/064032 A2 bekannt, wobei bei einigen Schriften die Signale der Funkstrecke nach dem sog. Bluetooth-Protokoll übertragen werden.

Das bekannte Bluetooth-Protokoll eignet sich für Funkverbindungen im Nahbereich bis zu etwa 10 m, arbeitet üblicherweise in einem Frequenzbereich von 2,402 GHz bis 2,480 GHz und führt 1600 mal pro Sekunde einen Frequenzwechsel in 79 Schritten zu je 1 MHz-Abstand durch (sog. Frequenz-Hopping-Verfahren). Vorteile dieses Verfahrens sind u.a. geringer Stromverbrauch, Übertragungssicherheit durch Störunanfälligkeit, so daß auch mehrere Geräte in einem Raum gemeinsam betrieben werden können. Im Gegensatz zur Übertragung mittels Infrarot-Signalen muß auch keine direkte Sichtverbindung zwischen Sender und Empfänger vorhanden sein. Aufgrund der sehr geringen Sendeleistung besteht auch nicht die Gefahr, daß andere elektronische Geräte gestört werden.

In der Medizintechnik und insbesondere in Operationssälen und auf Intensivstationen muß eine Vielzahl von Patientendaten laufend erfaßt und an eine Auswerteeinheit, wie z.B. einen Monitor, übertragen werden. Dies bedingt eine aufwendige "Verkabelung", die nicht nur aufwendig anzuschließen und zu verlegen ist, sondern auch Operateure und Klinikpersonal in ihrer Bewegungsfreiheit einschränkt, die Gefahr mit sich bringt, daß Kabel versehentlich getrennt, vertauscht oder gar geknickt werden und des weiteren gelegentlich Korrosionserscheinungen aufweisen, was zu gefährlichen Situationen für den Patienten führen kann. Insoweit bietet eine Funkübertragung vor allem mit dem Bluetooth-Verfahren wesentliche Vorteile. Gleichwohl müssen noch spezifische Probleme gelöst werden, um in einem Operationssaal oder auf einer Intensivstation eine Funkübertragung mittels Bluetooth-Protokoll von patientenseitigen Meßgeräten zu einer Auswerteeinheit durchführen zu können.

Dabei ist zu berücksichtigen, daß die patientenseitigen Meßgeräte üblicherweise am Patienten verbleiben, wenn er beispielsweise vom Operationssaal zu einer Wachstation oder zu einem Krankenhauszimmer gebracht wird, während die Überwachungseinheit normalerweise stationär im jeweiligen Raum, also z.B. dem OP-Saal, dem Wachraum oder dem Patientenzimmer, verbleibt. Daher müssen Sender und Empfänger mit verschiedenen Gegenstationen zusammenarbeiten können, gleichwohl darf es keinesfalls zu Verwechslungen kommen.

Zur Erhöhung der Patientensicherheit muß weiter der Zustand der Funkverbindung laufend überwacht und im Falle einer Verbindungsunterbrechung signalisiert werden. Gleiches gilt für die Überwachung der patientenseitigen Meßgeräte. Kurzzeitige Störungen der Signalübertragung dürfen keine spürbaren Auswirkungen haben. Auch soll das Gesamtsystem übersichtlich und leicht zu bedienen sein. Weitere Anforderungen sind der nachfolgenden Beschreibung zu entnehmen.

Zusammengefaßt ist es somit Aufgabe der Erfindung, die bekannte Patientenüberwachungsvorrichtung dahingehend zu verbessern, daß sie auch in besonders kritischen Einsatzfällen, wie Operationssaal, Intensiv- oder Wachstation, fehlerfrei und sicher arbeitet.

Diese Aufgabe wird durch die im Patentanspruch 1 angegebenen Merkmale gelöst. Vorteilhafte Ausgestaltungen und Weiterbildungen der Erfindung sind den Unteransprüchen zu entnehmen.

Das Grundprinzip der Erfindung beruht darauf, daß jeder patientenseitigen Sendeeinheit ein eindeutiger individueller Identifikationscode zugewiesen ist und die patientenferne Empfangseinheit erst dann eine Funkverbindung aufnimmt, wenn ihr dieser Identifikationscode eingegeben wurde. Nach einem Verbindungsabbruch muß der Identifikationscode an der patientenfernen Empfangseinheit wieder neu eingegeben werden. Ein Verbindungsabbruch erfolgt beispielsweise auch dann, wenn die patientenseitige Sendeeinheit räumlich aus der Reichweite der Funkverbindung gebracht wird, die in der Größenordnung von 10 m liegt. Wird also beispielsweise der Patient mit den Meßgeräten und der Sendeeinheit aus dem OP-Saal gebracht, so wird die Funkverbindung automatisch abgebrochen. Wird dann ein neuer Patient mit entsprechenden Meßgeräten und einer Sendeeinheit in den OP-Saal gebracht, so wird keine Funkverbindung mit der stationären Empfangseinheit aufgenommen, solange ihr nicht der neue Identifikationscode der neuen Sendeeinheit eingegeben wurde.

Selbstverständlich ist es auch möglich, die Funkverbindung durch aktives Betätigen einer Taste an der Sendeeinheit und/oder der Empfangseinheit zu beenden.

Zur Erhöhung der Übertragungssicherheit werden die patientenseitigen Meßdaten in der Sendeeinheit in einem Pufferspeicher zwischengespeichert und in vorbestimmten zeitlichen Intervallen als Datenpaket gesendet. Auch im Empfänger kann eine entsprechende Zwischenpufferung vorgesehen sein. Bei kurzfristigen Störungen der Funkstrecke kann der Empfänger die Aussendung eines entsprechenden Datenpaketes anfordern. Die Datenpakete können beispielsweise alle 100 ms ausgesandt werden, und zwar vorzugsweise in Form sog. Slip-Frames, die im Empfänger entpackt und in dem Datenpuffer zwischengespeichert werden.

Auch kann vorgesehen sein, daß ein und dasselbe Datenpaket zweimal hintereinander ausgesandt wird und im Empfänger die beiden aufeinanderfolgenden Datenpakete miteinander verglichen werden und nur dann als gültig angesehen werden, wenn sie identisch sind. Sind sie es nicht, so wird eine neue Datenaussendung angefordert. Eine hierdurch verursachte Verzögerung der Datenübertragung liegt in der Größenordnung von wenigen 100 ms (max. 500 ms) und ist bezüglich der zu überwachenden Patientendaten unkritisch.

Die Energieversorgung der patientenseitigen Geräte erfolgt wegen der geforderten Mobilität durch Batterien oder aufladbare Akkus. In der patientenseitigen Sendeeinheit wird der Ladezustand der Batterien oder Akkus laufend überwacht und an der Sendeeinheit angezeigt. Zusätzlich werden diese Daten auch an die Empfangseinheit übermittelt und dort ebenfalls angezeigt.

Zur Minimierung des Energieverbrauchs der patientenseitigen Sendeeinheit kann die Sendeleistung abgeschaltet werden, wenn keine Aussendung erfolgt.

Mit der Erfindung erreicht man eine Vielzahl von Vorteilen, die kurz zusammengefaßt folgendes beinhalten:

Aufgrund der Funkverbindung im OP-Saal werden störende Kabel, die den Arbeitsradius des medizinischen Personals einschränken oder stören, vermieden. Das durch die Kabel verursachte Sicherheitsrisiko für Patienten und Anwender durch Stolpern oder Hängenbleiben tritt nicht auf. Funktionsmängel am Kabel oder an Steckverbindern durch Beschädigung, falsche Befestigung, falsche Behandlung, Abnutzung oder Korrosion werden vermieden. Ein Kabelwirrwarr mit Gefahr der Verwechslungen, Unübersichtlichkeit und Behinderung des medizinischen Personals tritt nicht mehr auf. Der Abstand und die Position zwischen Patient und Auswerteeinheit ist nur durch die Reichweite der Funkstrecke beschränkt. Auch werden die Kosten, die sehr häufig durch defekte Kabel entstehen, vermieden.

Durch die eindeutige Zuordnung zwischen Sender und Empfänger und bei mehrkanaligen Meßstrecken auch deren eindeutige Zuordnung zwischen jeweiligem Meßgerät, wie z.B. Blutdrucksensor und entsprechendem Empfangskanal der Überwachungseinheit, wie z.B. einen Monitor, werden Verwechslungen, die sonst durch falsche Verkabelung entstehen, vermieden. Dabei ist die Bedienung und der Aufbau wesentlich vereinfacht. Weiter arbeitet die Verbindung störungsfrei und sicher, wird aber automatisch beim Verlassen des Sendebereichs abgebrochen und nicht automatisch wieder aufgenommen. Auch ist ein Abbruch der Verbindung durch den Anwender jederzeit möglich. Trotz der Verwendung von elektromagnetischen (Funk)-Wellen tritt durch die extrem geringe Sendeleistung keine Gefährdung der Patienten oder des Personals auf, und auch andere elektronischen Geräte werden nicht gestört.

Im folgenden wird die Erfindung anhand eines Ausführungsbeispiels im Zusammenhang mit der Zeichnung ausführlicher erläutert. Es zeigt:
- Fig. 1: schematisch einen grundlegenden Aufbau einer Vorrichtung zur Patientenüberwachung;
- Fig. 2: ein Blockschaltbild der Patientenüberwachungseinrichtung; und
- Fig. 3: ein Flußdiagramm des funktionellen Ablaufs eines Verbindungsaufbaus und einer Datenübertragung.

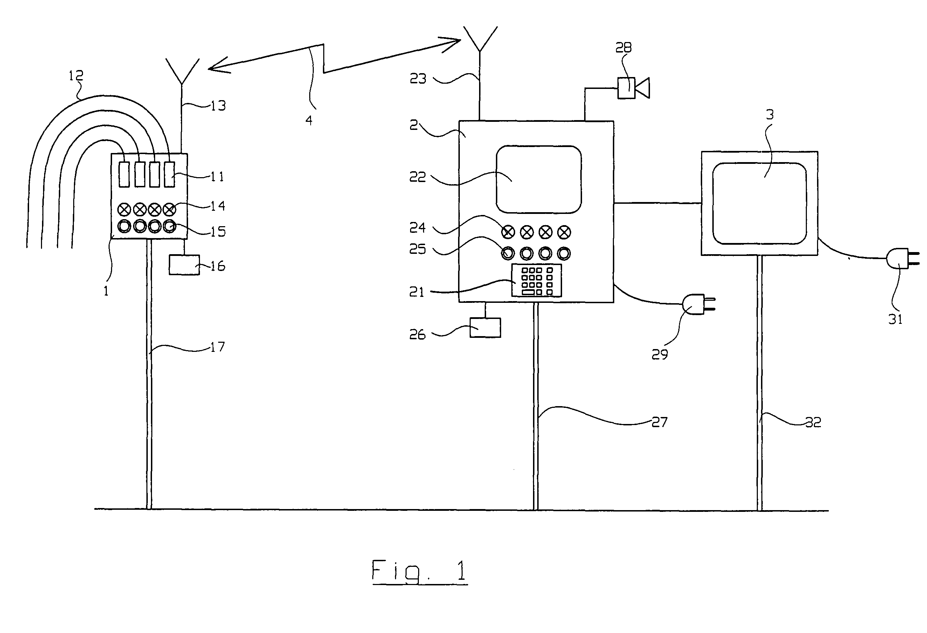

Fig. 1 zeigt den grundlegenden Aufbau der Patientenüberwachungsvorrichtung. Diese weist eine Sendeeinheit 1 auf, eine Empfangseinheit 2 und eine Auswerteeinheit 3, die mit der Empfangseinheit 2 verbunden ist. Die Sendeeinheit 1 und die Empfangseinheit 2 sind über eine bidirektionale Funkstrecke 4 miteinander verbunden. Bidirektional bedeutet in diesem Zusammenhang, daß sowohl Daten von der Sendeeinheit 1 zur Empfangseinheit 2 und auch von der Empfangseinheit 2 zur Sendeeinheit 1 übertragen werden können. Die Begriffe Sende- und Empfangseinheit sind hier so gewählt, daß sie sich auf die Übertragung von Meßdaten beziehen.

An der Sendeeinheit 1 sind ein oder mehrere Meßwandler 11 angebracht, die über Verbindungsleitungen 12 mit einem Patienten verbunden sind. Es kann sich hierbei beispielsweise um Druckmeßwandler handeln, die über die Leitungen 12 mit einem Blutgefäß, wie z.B. Vene oder Artherie, eines Patienten verbunden sind. Diese Druckmeßwandler wandeln einen Flüssigkeitsdruck in ein elektrisches Signal um, das, wie noch weiter unten beschrieben wird, nach einer Datenverarbeitung von der Sendeeinheit 1 über eine im Gehäuse oder auf einer Platine angebrachte, äußerlich nicht sichtbare Antenne 13 und die Funkstrecke 4 zu einer Antenne 23 der Empfangseinheit 3 übermittelt werden.

Die Meßwandler 11 können vorzugsweise, wie an sich bekannt, durch Aufstecken an der Sendeeinheit 1 befestigt und elektrisch mit dieser gekoppelt werden. Dies kann über an sich bekannte elektrische Kontakte an den Meßwandlern 11 und Gegenkontakte an der Sendeeinheit 1 erfolgen oder auch über kurze elektrische Kabel.

An der Sendeeinheit 1 sind mehrere Leuchtanzeigen 14, die vorzugsweise als lichtemittierende Dioden (LED's) ausgeführt sind, vorhanden sowie mehrere Tasten 15, auf die weiter unten noch eingegangen wird. Die Sendeeinheit 1 hat eine Spannungsversorgung 16, die vorzugsweise als aufladbarer Akku ausgebildet ist. Die Sendeeinheit 1 ist an einer Halterung 17 angebracht, die ein Stativstab sein kann oder eine sonstige Befestigungsvorrichtung, mit der die Sendeeinheit nahe dem Patienten, beispielsweise auch am Patientenbett, befestigt oder aufgestellt werden kann.

Die über die Funkstrecke 4 übertragenen Signale werden nach dem sog. Bluetooth-Protokoll übertragen, das eingangs erläutert wurde. Die Reichweite beträgt hierbei ca. 10 m und erfordert keine direkte "Sichtverbindung" zwischen den beiden Antennen 13 und 23. Die Antennen müssen daher auch nicht aufeinander ausgerichtet sein. Vielmehr strahlen und empfangen sie omnidirektional.

An der Empfangseinheit 2 ist eine Eingabeeinrichtung, wie z.B. eine Tastatur 21, angebracht, die im einfachsten Fall eine numerische Tastatur sein kann. Es kann aber auch eine erweiterte alphanumerische Tastatur sein. Weiter ist an einer Empfangseinheit 2 ein Display 22 angebracht. Darüber hinaus sind ein oder mehrere lichtemittierende Dioden 24 sowie mehrere elektrische Tasten 25 vorhanden. Die Energieversorgung der Empfangseinheit kann über einen externen Akku 26 und/oder über ein Netzkabel 29 erfolgen. Bei einer Stromversorgung über das Netz dient der Akku 26 als Puffer für eine Notstromversorgung.

Die Empfangseinheit 2 kann an einer Halterung 27, wie z.B. einem Stativstab, befestigt sein. Sie kann aber auch an einer Wand befestigt oder auf einem Tisch aufgestellt sein, da sie normalerweise stationär ist. Weiter weist die Empfangseinheit einen akustischen Signalgeber 28 auf, der bei bestimmten Betriebszuständen ein akustisches warnsignal erzeugt.

An die Empfangseinheit 2 ist eine Überwachungseinheit 3 angeschlossen, die ein herkömmlicher Monitor sein kann oder auch eine aufwendigere Einrichtung, wie z.B. ein Computer, der alle übermittelten Patientendaten protokolliert. Die Auswerteeinheit 3 wird üblicherweise mittels eines Netzkabels 31 vom Stromnetz mit Energie versorgt. Sie kann an einer Halterung 32, die beispielsweise ein Aufnahmepult ist, befestigt sein.

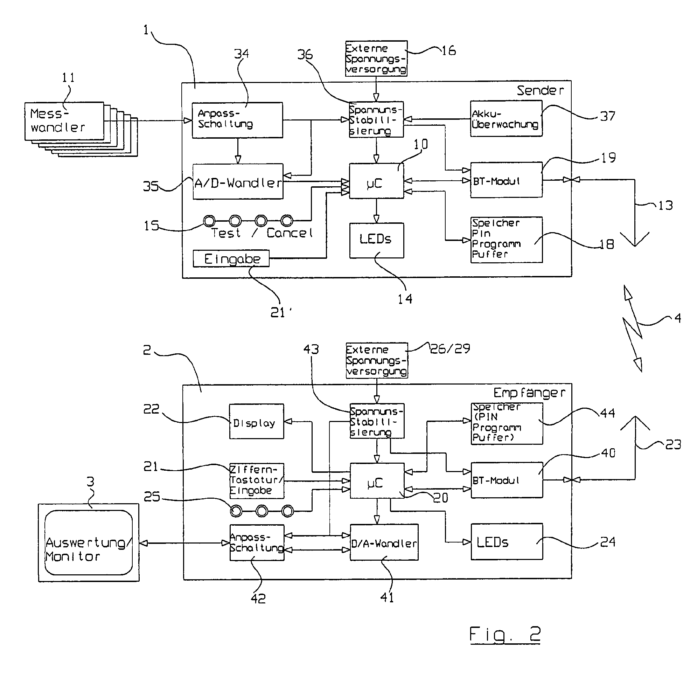

Fig. 2 zeigt ein Blockschaltbild der Sende- und Empfangseinheit 1 bzw. 2. An die Sendeeinheit 1 sind ein oder mehrere Meßwandler 11 angeschlossen. Kernstück der Sendeeinheit 1 ist ein Mikrocontroller 10, der über eine Anpaßschaltung 34 und einen Analog/Digitalwandler 35 elektrische Signale von den Meßwandlern 11 erhält. Dem Mikrocontroller 10 ist ein Speicher 18 zugeordnet, der u.a. einen eindeutigen Identifikationscode (im folgenden PIN genannt) enthält, der für jede Sendeeinheit 1 individuell und unverwechselbar vergeben wird. Weiter ist im Speicher 18 ein Programm für die Ablaufsteuerung des Mikrocontrollers 10 gespeichert. Schließlich dient der Speicher 18 auch als Datenpuffer, was weiter unten noch ausführlicher erläutert wird.

An den Mikrocontroller 10 ist ein sog. Bluetooth-Modul 19 angeschlossen, das die vom Mikrocontroller 10 aufbereiteten Meßdaten in das Bluetooth-Protokoll umsetzt und als Funksignale über die Antenne 13 über die Funkstrecke 4 sendet.

An den Mikrocontroller sind weiterhin die LED's 14 angeschlossen sowie die Tasten 15, die beispielsweise die Funktionen "Test" und "Verbindungsabbruch" (Cancel) auslösen.

Die externe Spannungsversorgung 16 ist mit einer Spannungsstabilisierung 36 verbunden, die ihrerseits den Mikrocontroller 10, das Bluetooth-Modul 19, die Anpaßschaltung 34 und den A/D-Wandler 35 mit stabilisierter Spannung versorgt. An die Spannungsstabilisierung ist auch eine Akkuüberwachung 37 angeschlossen, die den Ladezustand des Akkus 16 überwacht und regelmäßig über die Spannungsstabilisierung 36 an den Mikrocontroller 10 meldet.

Die Signalflußrichtung zwischen den einzelnen Baugruppen oder Funktionsteilen ist durch die jeweiligen Pfeile gekennzeichnet.

Die Empfangseinheit 2 hat als Kernstück ebenfalls einen Mikrocontroller 20, der an ein empfängerseitiges Bluetooth-Modul 40 angeschlossen ist, das seinerseits über die Antenne 23 mit der Funkstrecke 4 verbunden ist. Auch hier ist ein Speicher 44 an den Mikrocontroller 20 angeschlossen, der ebenfalls ein Programm enthält, als Pufferspeicher dient und auch einen Identifikationscode (PIN) enthält. Der PIN-Code der Empfangseinheit 2 ist frei änderbar und kann über eine Eingabeeinrichtung 21, wie z.B. eine Zifferntastatur 21, die an den Mikrocontroller 20 angeschlossen ist, eingegeben werden. Damit eine Funkverbindung zwischen Sender und Empfänger aufgebaut werden kann, muß am Empfänger die identische PIN der Sendeeinheit 1 eingegeben werden. Erst dann kann ein Verbindungsaufbau begonnen werden, was weiter unten noch ausführlicher erläutert wird. An den Mikrocontroller 20 sind weiterhin die erwähnten LED's 24, die Tasten 25 und das Display 22 angeschlossen. Weiter ist an den Mikrocontroller 20 ein Digital/Analog-Wandler 41 angeschlossen, der über eine Anpaßschaltung 42 mit der Auswerteeinheit 3 verbunden ist. Schließlich ist auch hier eine Spannungsstabilisierungsschaltung 43 vorgesehen, die mit der externen Versorgungsspannung, also dem Akku 26 und/oder dem Netzkabel 29 verbunden ist. Auch hier ist die jeweilige Signalflußrichtung durch Pfeile gekennzeichnet.

Die Verbindung zwischen der Auswerteeinheit 3 und der Empfangseinheit 2 kann - wie durch die Pfeile dargestellt - bidirektional sein, so daß beispielsweise von der Auswerteeinheit 3 auch Signale, wie z.B. Datenanforderung oder Warnsignale, zur Empfangseinheit 2 übertragen werden können.

Im Zusammenhang mit dem Blockschaltbild der Fig. 2 sei darauf hingewiesen, daß die Darstellung Funktionseinheiten zeichnet, die nicht unbedingt als separate Baugruppen implementiert sein müssen. Beispielsweise kann die A/D- und die D/A-Wandlung vom Mikroprozessor durchgeführt werden. Ebenso kann die Akkuüberwachung und auch die Regelung der Spannungsstabilisierung programmgesteuert vom jeweiligen Mikrocontroller 10 bzw. 20 übernommen werden.

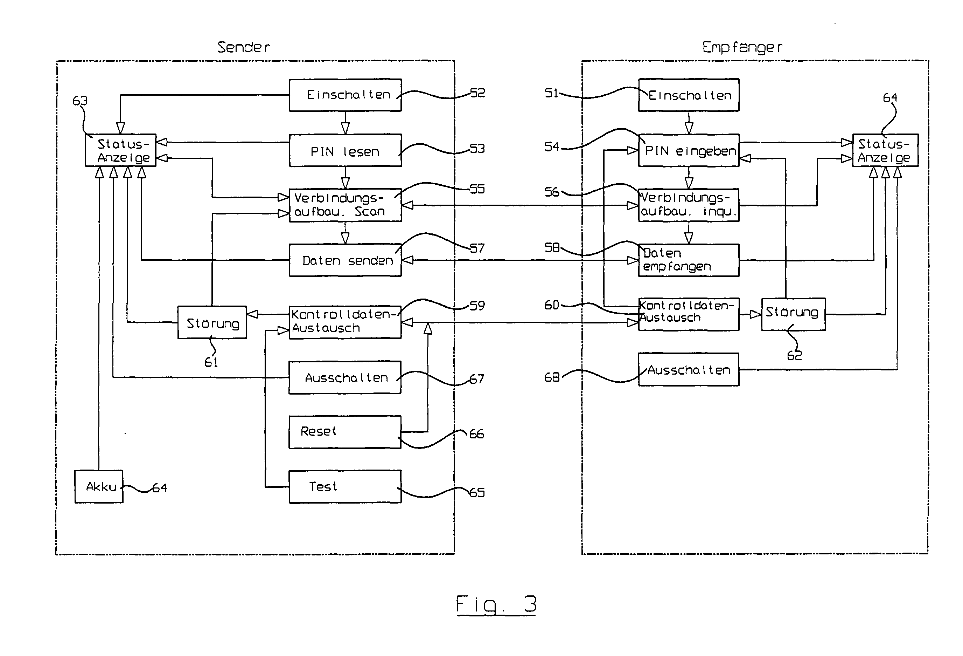

Im Zusammenhang mit Fig. 3 wird die Funktionsweise der Patientenüberwachungsvorrichtung erläutert. Es wird davon ausgegangen, daß Sende- und Empfangseinheit 1 und 2 innerhalb der Reichweite der Funkstrecke 4 plaziert sind und beide Einheiten mit elektrischer Energie versorgt sind.

Der Empfänger sei eingeschaltet, beispielsweise durch Einschalten der Energieversorgung (Block 51), und der Mikrocontroller mit seinem Programm geladen.

Auch der Sender sei eingeschaltet (Block 52), was ebenfalls durch Anschließen der Stromversorgung, einen Schalter oder auch durch Anschließen mindestens eines Meßwandlers 11 erfolgen kann. Die Sendeeinheit hat - wie erwähnt - in ihrem Speicher einen eindeutigen Identifikationscode (PIN), der nach dem Einschalten des Senders gelesen wird (Block 53). Dieser Code, der vorzugsweise als numerischer Zahlencode vorliegt, ist deutlich sichtbar an der Sendeeinheit angebracht. Dieser Code muß an der Empfangseinheit über die Eingabeeinrichtung 21 eingegeben werden (Block 54). Ist diese eine Tastatur, so erfolgt die Eingabe von Hand. Erst wenn dies erfolgt ist, wird zwischen Sender und Empfänger ein Verbindungsaufbau gestartet. Beim Verbindungsaufbau (Block 55) ist der Sender in der passiven Rolle als Empfänger und wartet, ob ein Verbindungswunsch an ihn gerichtet wird. Dies hat den Vorteil, daß der Sender in dieser Phase weniger Energie verbraucht, keine Störungen im Frequenzband verursacht und nicht aktiv sendet. Die aktive Rolle beim Verbindungsaufbau übernimmt der Empfänger (Block 56). Sobald dort eine PIN eingegeben wurde, fängt er an, nach Gegenstationen in seiner Reichweite zu suchen, was durch ein sog. Inquiry-Signal erfolgt. Es findet dann der für das Bluetooth-Protokoll übliche "Pairing" statt, so bald ein aktivierter Sender (der zu diesem Zeitpunkt als Empfänger arbeitet) gefunden wurde. Sodann wird über einen Austausch der wechselseitigen PIN's der Verbindungsaufbau durchgeführt. Stimmen die beiden PIN's von Sender und Empfänger überein, so wird die Verbindung hergestellt, stimmen die PIN's nicht überein, fallen beide Seiten wieder in die vorherige Rolle zurück, d.h. der Sender wird passiv "lauschen" und der Empfänger aktiv suchen, da möglicherweise ein falscher aber ebenfalls aktivierter Sender kontaktiert wurde.

Ist ein Verbindungsaufbau erfolgreich durchgeführt worden, so ist ein weiterer Verbindungsaufbau mit anderen Sendern oder Empfängern gesperrt.

Nach erfolgreichem Verbindungsaufbau werden Nutzdaten vom Sender ausgestrahlt (Block 57) und vom Empfänger empfangen (Block 58). Zusätzlich werden Kontrolldaten ausgetauscht (Blöcke 59 und 60). Kontrolldaten können beispielsweise Störungsmeldungen (Blöcke 61 und 62) enthalten, die am Sender und/oder Empfänger als Statusanzeige (Blöcke 63 und 64) an den jeweiligen LED's (14 und 24 in Fig. 2) angezeigt werden. Auch normale Betriebszustände, wie z.B. Akku in Ordnung (Block 64), Meßwandler angeschlossen, Funkverbindung steht etc., können dort zur Anzeige gebracht werden. Durch Drücken einer der Tasten 15 am Sender (Block 65) kann auch eine Testroutine für den jeweiligen Meßwandler ablaufen, beispielsweise indem bei einem Druckmeßwandler in bekannter Weise ein definiertes Sollwertsignal (üblich ist 100 mm/Hg) ausgelöst wird, das im Empfänger 2 ausgewertet wird und an einer der LED' s 24 und/oder am Monitor 3 zur Anzeige gebracht wird. Hierdurch kann eine direkte Verbindung überprüft werden.

Einer der Tasten 15 des Senders kann auch eine Resetfunktion (Block 66) zugewiesen sein. Auch können den Tasten 15 am Sender und der Tastatur 21 oder den Tasten 25 am Empfänger eine Ausschaltfunktion (Blöcke 67 und 68) zugewiesen sein, mit denen die Funkverbindung beendet wird. Nach Drücken einer dieser Tasten findet kein neuer Verbindungsaufbau statt, solange nicht am Empfänger wiederum eine PIN eingegeben wurde.

Bei einer Störung der Funkverbindung wird dies auf den Statusanzeigen (Blöcke 63 und 64) und konkret an den LED's 14 und 24 zur Anzeige gebracht. Zusätzlich kann ein akustischer Alarm (Signalgeber 28 in Fig. 1) ausgelöst werden.

Dauert die Störung der Funkstrecke kürzer als eine vorgegebene Zeit, beispielsweise 10 Sekunden, so wird ein neuer automatischer Verbindungsaufbau gestartet. Dauert die Störung länger als diese Zeit, so gehen Sender und Empfänger in ihren Initialisierungszustand und ein neuer Verbindungsaufbau kann erst wieder nach Neueingabe einer PIN am Empfänger (Block 54) erfolgen. Dadurch wird das Verwechslungsrisiko verringert. Wird beispielsweise der Sender aus dem Empfangsbereich gebracht, beispielsweise indem der Patient aus dem OP-Saal verbracht wird, so kann ein neuer Verbindungsaufbau mit demselben oder einem anderen Sender nur nach Neueingabe der PIN stattfinden.

Ein manuelles Abbrechen der Verbindung kann durch Drücken einer Ausschalttaste (Blöcke 67 und 68) oder einer Reset-Taste (Block 66) eingeleitet werden, wodurch die PIN im Empfänger gelöscht wird. Auch wird die Funkverbindung dann abgebrochen, wenn alle Meßwandler 11 vom Sender getrennt sind.

Am Empfänger wird zu jeder Zeit der aktuelle Verbindungszustand und beim verbundenen Sender dessen Akkuzustand überprüft und mittels LED's und/oder am Display 22 dargestellt. Zusätzlich werden auf dem Display Meldungen im Klartext dargestellt, wie z.B. falscher PIN, PIN-Eingabe etc. Bei Bestehen der Verbindung zu einem Meßwandler können auch Statusmeldungen dieses Meßwandlers im Klartext ausgegeben werden. Auf der Senderseite genügt es für die Praxis, an den LED's den Akkuzustand und bei Verwendung von mehreren Meßwandlern die Betriebsbereitschaft der jeweiligen Meßkanäle anzuzeigen. Der Akkuzustand sollte zweckmäßigerweise zusätzlich - bei vordefiniertem kritischen Zustand - auch akustisch signalisiert werden, z.B. durch einen intermittierenden oder intervallmäßigen Alarmton.

Die Statusanzeige der LED's kann auch mit mehrfarbigen LED's durchgeführt werden, beispielsweise mit den Farben rot und grün, wobei Zustände zusätzlich durch ein Blinken angezeigt werden können. Zweckmäßigerweise sind die Statusan- , zeigen am Sender und Empfänger identisch aufgebaut, um den Benutzer nicht durch unterschiedliche Anzeigen zu verwirren. Folgende Zustände werden durch die LED's signalisiert:

Bootvorgang, Bereitmodus, Verbindungsaufbau, kein Verbindungsaufbau möglich, Datenübertragung und Akku o.k., Datenübertragung und Akku kritisch, temporäre Verbindungsstörung, Verbindung durch Störung abgebrochen, keine Verbindung, Akkuzustand niedrig.

Zusätzlich können am Sender weitere LED's angebracht sein entsprechend der Anzahl der anschließbaren Meßwandler. Durch Ein- oder Ausschalten der zugeordneten LED kann angezeigt werden, ob der entsprechende Meßwandler angeschlossen oder nicht angeschlossen ist.

Auf dem Display des Empfängers können zusätzlich im Klartext folgende Angaben gemacht werden, wobei über die Tastatur am Empfänger auch eine Sprache (z.B. deutsch, englisch, französisch etc.) gewählt werden kann:

Bootvorgang, betriebsbereit, PIN-Eingabe, verbindungssuche, Datenempfang, Information zum Verbindungsstatus, Verbindungsstörung, Verbindungsabbruch, Akkuzustand niedrig, Problem beim Verbindungsaufbau, keine Gegenstelle gefunden.

Der akustische Signalgeber 28 am Empfänger und/oder am Sender kann ein Summer sein, der bei kritischem Akkuzustand einen Warnton erzeugt.

Für viele Anwendungszwecke wird die Tastatur 21 am Empfänger als Zifferntastatur mit wenigen zusätzlichen Funktionstasten ausreichend sein. Über diese Tastatur erfolgt die PIN-Eingabe, ggf. eine PIN-Korrektur, eine PIN-Bestätigung und eine Konfiguration der Sprache.

Die Reset- oder Cancel-Taste ist vorzugsweise besonders markiert hervorgehoben und am Sender und Empfänger an gleicher oder geeignet hervorgehobener Position angeordnet.

Die Festlegung der PIN erfolgt vorzugsweise für jedes Sendermodul. In dieser PIN kann auch der Name des Krankenhauses codiert enthalten sein, so daß man bei der üblicherweise selbst in Großkliniken vorhandenen Stückzahl normalerweise mit einer fünfstelligen PIN auskommt. Nach einer Variante der Erfindung kann die im Sender fest eingespeicherte PIN auch zusätzlich auf dem Gehäuse des Senders als Strichcode aufgedruckt sein, wobei dann der Empfänger ein StrichcodeLesegerät hat, um die PIN einzugeben. Hierdurch werden eventuelle Fehler beim Eingeben der PIN über eine Tastatur vermieden. Somit kann nach einer Alternative der Erfindung die Eingabeeinheit 21, 21' am Sender und am Empfänger auch als Lesegerät für eine Magnet- oder Chipkarte ausgebildet sein, auf welcher die PIN für Sender und Empfänger gespeichert ist und jeweils vor Zusammenstellen eines Paares von Sender und Empfänger eingegeben wird. Die Eingabeeinheit 21 am Empfänger 2 kann auch ein Lesegerät für einen Strichcode sein, der auf dem Gehäuse der Sendeeinheit 1 aufgedruckt ist und von dem der Empfangseinheit 2 zugeordneten Strichcodelesegerät abgelesen wird. Da die PIN bei Unterbrechung der Funkverbindung für eine vorgegebene Zeitdauer gelöscht wird (z.B. nach 10 Sekunden), besteht auch nicht die Gefahr, daß beispielsweise ein neu in einen Operationssaal verbrachter Sender noch eine "alte" PIN gespeichert hat, die mit der am Empfänger gespeicherten PIN übereinstimmt.

Störungen auf der Funkstrecke werden prinzipiell vom Bluetooth-System erkannt und veranlassen normalerweise einen Neuaufbau der Funkverbindung. Bei der Erfindung ist allerdings vorgesehen, daß dies nur für Unterbrechungen innerhalb einer bestimmten Zeitdauer stattfindet. Bei längeren Unterbrechungen soll eine automatische Neuaufnahme nicht möglich sein.

Kurze Unterbrechungen haben zur Folge, daß bei der Einspeisung von digitalen Eingangswerten im D/A-Wandler des Empfängers Unterbrechungen entstehen, was wiederum zur Folge hat, daß die Wandlerschritte nicht mehr äquidistant sein können. Aus diesem Grunde wird eine Pufferung der Sensordaten im Speicher 44 des Empfängers vorgenommen, wodurch die Stabilität der Datenübertragung erhöht wird. Diese Stabilität erkauft man sich zwar durch eine Vergrößerung einer Verzögerungszeit, die umso größer ist, je größer die zwischengepufferte Datenmenge ist.

Da die Datenrate über die Funkstrecke (z.B. 721 kBit/s) wesentlich größer ist als die benötigte Datenrate der Meßwandler (ca. 9,6 k), kann das Bluetooth-Modul 19 des Senders zur Energieeinsparung zwischenzeitlich abgeschaltet werden. Nach dem Anschalten des senderseitigen Bluetooth-Moduls 19 können dann alle angefallenen Werte, die im Sender zwischengespeichert, d.h. gepuffert wurden, übertragen werden. Die dadurch eintretende Verzögerung ist hier unkritisch und kann beispielsweise auf angemessene Werte von bis zu einigen Sekunden festgelegt werden.

Die Meßwerte der Meßwandler werden bei einer Blutdruckmessung mit einer Abtastrate von 200 Hz und einer Auflösung von 12 Bit gewandelt, woraus sich eine Datenrate von 2400 Bit/s pro Meßkanal ergibt. Die zu übertragenden Daten werden vom Mikrocontroller in einem vorgegebenen Übertragungsformat als sog. Frame gepackt und paketweise übertragen. Jedes Datenpaket kann eine Sequenznummer enthalten, die fortlaufend hochgezählt wird. Zusätzlich zu den digital gewandelten Meßdaten können in ein Datenpaket auch Kontrolldaten eingefügt werden, beispielsweise über Akkuzustand, Testknopf gedrückt, Authentifizierungsanfrage, Authentifizierungsantwort, Störung sowie Identifizierung des jeweiligen Meßwandlers.

## Patentansprüche

1. Patientenüberwachungsvorrichtung mit einer patientenseitigen Sendeeinheit (1), an die mindestens ein patientenseitiger Meßwandler (11) angeschlossen ist und die Meßdaten über eine Funkstrecke (4) an eine Empfangseinheit (2) sendet,
**dadurch gekennzeichnet,**
**daß** in der Sendeeinheit (1) ein eindeutiger Identifikationscode gespeichert ist,
**daß** die Empfangseinheit (2) eine Eingabeeinrichtung (21) aufweist, mittels der ein Identifikationscode eingebbar ist, wobei die Empfangseinheit einen Speicher (44) aufweist, in dem der eingegebene Identifikationscode speicherbar ist, und
**daß** ein Verbindungsaufbau zwischen Sende- und Empfangseinrichtung (1, 2) nur dann erfolgt, wenn die Codes in der Sende- und Empfangseinheit (1, 2) identisch sind.

2. Patientenüberwachungsvorrichtung nach Anspruch 1, **dadurch gekennzeichnet,**
**daß** nach einer Störung der Funkverbindung zwischen Sende- und Empfangseinheit (1, 2) für länger als eine vorgegebene Zeitdauer der in der Empfangseinheit (1) gespeicherte Identifikationscode gelöscht wird und ein erneuter Verbindungsaufbau erst nach Neueingabe des Identifikationscodes an der Empfangseinheit (1) möglich ist.

3. Patientenüberwachungsvorrichtung nach Anspruch 1 oder 2, **dadurch gekennzeichnet,**
**daß** die Sende- und Empfangseinheit (1, 2) je ein Bluetooth-Modul (19, 40) aufweisen und daß die Funkverbindung zwischen Sende- und Empfangseinheit (1, 2) nach dem Bluetooth-Protokoll erfolgt.

4. Patientenüberwachungsvorrichtung nach einem der Ansprüche 1 bis 3, **dadurch gekennzeichnet,**
**daß** die Sende- und Empfangseinheit (1, 2) je einen Mikroprozessor (10, 20) und mindestens einen daran angeschlossenen Speicher (37, 44) aufweisen und daß die Mikroprozessoren (10, 20) durch in den Speichern (37, 44) gespeicherte Programme den Ablauf des Aufbaus der Funkverbindung derart steuern, daß die Empfangseinheit (2) aktiv Anforderungssignale aussendet, während die Sendeeinheit (1) passiv auf den Empfang von Anforderungssignalen wartet und erst nach einem solchen Empfang in aktiven Sendebetrieb geht.

5. Patientenüberwachungsvorrichtung nach einem der Ansprüche 1 bis 4, **dadurch gekennzeichnet,**
**daß** von dem mindestens einen Meßwandler (11) aufgenommenen Daten im Speicher (18) der Sendeeinheit (1) zwischengespeichert werden und in vorgegebenen zeitlichen Abständen paketweise an die Empfangseinheit (2) gesendet werden.

6. Patientenüberwachungsvorrichtung nach Anspruch 5, **dadurch gekennzeichnet,**
**daß** das Bluetooth-Modul (19) der Sendeeinheit (1) in den Zeitintervallen, in denen keine Datenpakete gesendet werden, bezüglich Aussendungen deaktiviert ist.

7. Patientenüberwachungsvorrichtung nach Anspruch 5 oder 6, **dadurch gekennzeichnet,**
**daß** die Datenpakete zusätzlich zu den Meßdaten Kontrolldaten enthalten.

8. Patientenüberwachungsvorrichtung nach Anspruch 7, **dadurch gekennzeichnet,**
**daß** die Sendeeinheit (1) eine Überwachungsschaltung (37) aufweist, die den Ladezustand eines Akkus (16) der Sendeeinheit (1) überwacht und entsprechende Akkuüberwachungsdaten erzeugt, und daß die Akkuüberwachungsdaten als Kontrolldaten an die Empfangseinheit (2) übertragen werden.

9. Patientenüberwachungsvorrichtung nach einem der Ansprüche 1 bis 8, **dadurch gekennzeichnet,**
**daß** die Sende- und die Empfangseinheit (1, 2) identische Leuchtanzeigen (14, 24) aufweisen, mittels denen vorgegebene Betriebszustände signalisierbar sind.

10. Patientenüberwachungsvorrichtung nach einem der Ansprüche 1 bis 9, **dadurch gekennzeichnet,**
**daß** die Sende- und Empfangseinheit (1, 2) je mindestens eine identische Taste aufweisen, mit der die Funkverbindung unterbrechbar und der gespeicherte Identifikationscode in der Empfangseinheit löschbar ist.

11. Patientenüberwachungsvorrichtung nach Anspruch 1, **dadurch gekennzeichnet,**
**daß** der eindeutige Identifikationscode in der Sendeeinheit (1) werksseitig fest vorgegeben ist und daß die Eingabeeinrichtung der Empfangseinheit (2) eine Tastatur (21) ist.

12. Patientenüberwachungseinrichtung nach Anspruch 1, **dadurch gekennzeichnet,**
**daß** die Sendeeinheit (1) und die Empfangseinheit (2) je eine Eingabeeinrichtung (21, 21') aufweisen zum Einlesen des Identifikationscodes von einem gemeinsamen Datenträger, wie z.B. einer Magnet- oder Chip-Karte.
